# EUROPÄISCHE PATENTANMELDUNG

(11) **EP 2 853 223 A1**
(43) Veröffentlichungstag der Anmeldung: **01.04.2015**
(21) Anmeldenummer: 14182883.0
(22) Anmeldetag: 29.08.2014
(51) Int. Cl.: A61B 19/00

(54) **Navigationsaufsatz und Verfahren zu dessen Anwendung**

(30) Priorität: 30.08.2013 DE 102013109486
(71) Anmelder: SurgicEye GmbH, 81671 München (DE)
(72) Erfinder: Friebe, Michael, 45657 Recklinghausen (DE)
(74) Vertreter: Zimmermann & Partner

(57) **Zusammenfassung**

Navigationsverfahren und Navigationsaufsatz (1) für ein manuell geführtes Instrument (20, 22), das zur chirurgischen und/oder therapeutischen Behandlung eines Körpers (5) und/oder zur Durchführung eines Diagnostizierverfahrens am Körper (5) bestimmt ist, umfassend: ein Befestigungsmittel, das angepasst ist, mit dem Instrument (20, 22) starr verbindbar zu sein, und zumindest eine Bildaufnahmeeinheit (4, 41, 42, 43), umfassend eine Vorrichtung zur Ermittlung eines Abstandswertes zwischen dem Navigationsaufsatz (1) und einer Oberfläche des Körpers (5), wobei zumindest drei Abstandswerte zur Darstellung mit einer Bildausgabeeinheit einer Position (x, y, z, t) des Instruments (20, 22) und/oder eines in physischen Kontakt mit dem Körper tretenden Vorderabschnitts (21) des Instruments (20, 22) bezogen auf den Körper (5) oder einen Bestandteil desselben verwendbar sind.

## Beschreibung

Diese Offenbarung bezieht sich auf medizintechnische Geräte und Verfahren zu deren Anwendung. Insbesondere bezieht sie sich auf Geräte und Verfahren für die präzise Handhabung einer manuell oder robotisch geführten Vorrichtung, wie insbesondere eines chirurgischen Instruments, eines Ultraschallkopfes oder eines anderen diagnostischen Systems, z.B. einer Gammasonde, einer Gammakamera, einer Fluoreszenzkamera und/oder einer Bestrahlungsquelle.

### TECHNISCHER HINTERGRUND

Qualitativ hochwertige Bilderzeugung und/oder die Nachverfolgung von Operationsinstrumenten, z.B. auf einem Bildschirm, ist von großem Interesse für einen weiten Bereich von Anwendungen. Insbesondere im medizinischen Bereich, wo die Gesundheit eines Patienten davon abhängen kann, ist eine bestmögliche Bilderzeugung und/oder Ortsbestimmung von Instrumenten und Sonden erforderlich, beispielsweise als Basis für Operationen am Patienten.

Insbesondere das Tracking (die Nachverfolgung) von handgehaltenen Sonden als übliche Diagnosegeräte, insbesondere während eines chirurgischen Eingriffs, sowie Trackingsysteme (Nachverfolgungssysteme) zur Bestimmung der Position und Orientierung von Operationsinstrumenten und Bildgebungsgeräten sind bekannt.

Dabei erschwert die häufig komplexe Anordnung verschiedener technischer Hilfsmittel, insbesondere von Haken, Klemmen, Clips, Klammern, Schläuchen, Kathetern, Kapillaren, Sonden, Elektroden oder anderen Instrumenten und Hilfsmitteln, sowie die Präsenz von Händen und Armen der an der Bildgebung / Therapie beteiligten Personen im Operationsfeld eine genaue Positionierung von Instrumenten und Sonden, da der unmittelbare Sichtkontakt zum jeweiligen Anwendungsareal erschwert oder verhindert sein kann. Ebenso können Probleme dadurch entstehen, dass ein Instrument oder eine Sonde zeitweise nicht im Blickfeld extern angebrachter Kamerasysteme ist, und damit zumindest zeitweise auch nicht kontrolliert bzw. nachverfolgt werden kann. Zudem können elektromagnetische Bildstörungen durch Interferenz einzelner Systemkomponenten entstehen.

Vor dem Hintergrund dieser und anderer Probleme ist die Aufgabe der vorliegenden Erfindung, eine verbesserte Vorrichtung und ein Verfahren bereitzustellen.

### ZUSAMMENFASSUNG

Vor diesem Hintergrund werden ein Navigationsaufsatz für ein manuell geführtes Instrument gemäß Anspruch 1 und ein Navigationsverfahren gemäß Anspruch 6 vorgeschlagen.

Insbesondere betreffen Ausführungsformen der Erfindung eine mit einer Kameraanordnung versehene Kappe, die auf eine vom Körper wegweisende Seite des betreffenden Instruments, typischerweise auf einen Handgriff des Instruments, aufgesetzt wird, mit dem Instrument arretiert wird und eine bildliche Darstellung des Instruments in einer auf andere Art verwirklichten Visualisierung des Körpers oder eines Körperabschnitts des Patienten ermöglicht, ohne die auf andere Art und Weise verwirklichte Visualisierung zu beeinträchtigen, etwa durch elektromagnetische Bildstörungen. Die in Ausführungsformen beispielsweise auf den Griff des betreffenden Instruments fest aufsetzbare Kappe liefert über die Erfassung eines Abstands und Winkels zu zumindest einer auf dem Körper angebrachten Markierung in Wechselwirkung mit einer Verrechnungseinheit und einer Ausgabeeinheit aktuelle Raumkoordinaten des Instruments, sowie einer damit verbundenen Zeiteinheit (4D), insbesondere bezogen auf seinen vorderen Abschnitt, in Relation zu dem mit dem Markierungselement versehenen Körper und/oder ermöglicht eine schematische Darstellung des Instruments in einer unabhängig realisierten Aufnahme des Körpers oder eines betreffenden Körperabschnitts. Das ermöglicht einem Operator auch ohne unmittelbaren visuellen Kontakt zum vorderen Ende des Instruments dessen präzise Handhabung auf Grund des unabhängig bereitgestellten Abbildes des Körpers bzw. Körperabschnitts. Die Navigationsvorrichtung ist neben der diagnostischen Überlagerung von mehreren Verfahren (z.B. Ultraschall und Gammakamera) auch vorteilhaft für den Bereich der minimalinvasiven Chirurgie und der bildgesteuerten Intervention/Therapie nutzbar.

Der Navigationsaufsatz gemäß Ausführungsbeispielen ist zur visuell, über eine Bildwiedergabeeinheit, kontrollierten Handhabung und Darstellung des Instruments am oder im Körper eines Patienten angepasst.

Gemäß einer Ausführungsform wird ein Navigationsaufsatz vorgeschlagen, der an einem manuell oder robotisch geführten Instrument, das zur chirurgischen oder therapeutischen Behandlung eines Körpers und/oder zur Durchführung eines Diagnostizierverfahrens am Körper bestimmt ist, befestigbar bzw. arretierbar ist. Unter einem Körper wird hierbei der Körper eines menschlichen oder tierischen Organismus verstanden, sowie bei Anwendungen außerhalb der medizinischen Diagnostik/Therapie der jeweilig zu bearbeitende nicht-menschliche Körper (beispielsweise bei Materialprüfungen von Bauteilen / Systemen). Der Navigationsaufsatz ist mit dem Instrument starr verbindbar und hat weiterhin zumindest eine Bildaufnahmeeinheit, die eine Vorrichtung zur Ermittlung eines Abstands- und optional eines Winkelwertes zwischen dem Navigationsaufsatz und einer Oberfläche des Körpers bzw. einer Körperachse umfasst. Aus zumindest drei ermittelten Abstandswerten des Navigationsaufsatzes zur Oberfläche des Körpers ist eine Position (x, y, z) des Instruments zum gegebenen Zeitpunkt t und/oder eines vorderen Abschnitts des Instruments bezogen auf den Körper oder einen Bestandteil desselben zum gegebenen Zeitpunkt t ermittelbar. Das gleiche ist auch der Fall, wenn es an einem Punkt am Körper zumindest drei unterschiedliche Marker gibt, die von dem Navigationsaufsatz an einer Stelle erkannt werden können.

Vorteilhafterweise ist in Ausführungsformen die ermittelte Position mit Hilfe einer Ausgabeeinheit darstellbar, so dass die Führung des Instruments durch einen Operator erleichtert wird, indem die Position des Instruments z.B. auf einem vorab aufgenommenen Datenbild gezeigt wird bzw. die aufgenommen Daten des Instruments mit einem Datenbild überlagert werden bzw. die aufgenommenen Daten des Instruments ein eigenständiges 3D Bild generieren bzw. eine genaue Positionierung des Instruments in einem vorab aufgenommenen Datenbild ermöglichen, zu dem die Navigationsdaten referenziert werden. Weitere Vorteile ergeben sich insbesondere dann, wenn der Bediener des Instruments, d.h. der Operator, den Körper oder Körperabschnitt, welcher einer Behandlung oder einer eingehenderen Diagnose mit dem Instrument unterzogen wird oder werden soll, ohnehin in Form einer bildlichen Darstellung sieht, diese Art der Darstellung jedoch das jeweilige Instrument nicht korrekt abbildet oder dessen korrekte Erfassung mit derselben Technik die bisherige Bildgüte beeinträchtigen würde. Beispielsweise erlaubt es der vorgeschlagene Navigationsaufsatz einerseits ohne eine zusätzliche Belastung des Körpers durch zusätzliche Kontrastmittel oder andere Markierungshilfen und andererseits ohne eine elektromagnetische Beeinträchtigung der Abbildungsgüte der bestehenden Abbildes auch das Instrument darstellen zu können. Aus jeweils aktuellen Positionswerten des Navigationsaufsatzes - die ggf. stets in einem sehr kurzen Zeittakt erneuert werden - wird eine bildliche Darstellung des Instruments oder seines vorderen Abschnitts in ein gemäß einem anderen Darstellungsprinzip erzeugtes Abbild eingespielt, diesem überlagert, oder darin sichtbar gemacht. Daraus ergibt sich der große Vorteil, dem Operator eine genaue Instrumentennavigation im jeweiligen Körper, Körperabschnitt, oder Gewebe zu ermöglichen.

Gemäß einer weiteren Ausführungsform wird ein Navigationsaufsatz vorgeschlagen, wobei der Navigationsaufsatz eine Ausnehmung umfasst, die so angepasst ist, dass sie reversibel und zumindest abschnittsweise formschlüssig mit einem rückwärtigen Abschnitt, beispielsweise einem Griff-Ende des Instruments, verbindbar ist, wobei die zumindest eine Bildaufnahmeeinheit benachbart zur Ausnehmung angeordnet ist. Beispielsweise kann die Ausnehmung so ausgestaltet sein, dass sie in einer bestimmten Orientierung zum Instrument einrastet, der Aufsatz also abschnittsweise formschlüssig mit dem Instrument verbindbar ist.

Ein Vorteil dieser Merkmale ergibt sich daraus, dass ein und dieselbe Navigationseinheit universell für verschiedene Instrumente einsetzbar ist. Bei Kenntnis instrumentenspezifischer Parameter (Länge, evtl. Krümmungen etc.) können mit Hilfe des Navigationsaufsatzes ermittelte Positionswerte kalibriert bzw. auf die Position eines Vorderabschnitts des Instruments bezogen werden.

Gemäß einer weiteren Ausführungsform wird ein Navigationsaufsatz vorgeschlagen, der drei Bildaufnahmeeinheiten umfasst, wobei die Bildaufnahmeeinheiten jeweils angepasst sind, einen Abstandswert zu mindestens einem Markierungselement auf der Oberfläche des Körpers und optional einen Wert einer axialen Rotation relativ zum Markierungselement zu erfassen, wenn sich das Markierungselement im Erfassungsbereich der Bildaufnahmeeinheit befindet.

Vorteile dieser Ausführungsform ergeben sich aus der Möglichkeit einen Verrechnungsalgorithmus erhaltener Abstandswerte mit Hilfe standardisierter Markierungselemente zu standardisieren. Damit wird die Zuverlässigkeit errechneter Positionswerte bzw. Raumkoordinaten erhöht.

Gemäß einer weiteren Ausführungsform wird ein Navigationsaufsatz vorgeschlagen, wobei der Navigationsaufsatz angepasst ist, ermittelte Werte an eine Auswerteeinheit zu übermitteln, die in Verbindung mit einer Ausgabeeinheit steht, wobei die Auswerteeinheit so hergerichtet ist, dass eine Darstellung des Instruments und/oder zumindest seines vorderen Abschnitts ermöglicht wird. Die erhaltene Abbildung kann ebenso in ein bestehendes Abbild des Körpers oder dessen Bestandteil eingebettet werden.

Vorteile bestehen in der verbesserten Durchführbarkeit von Inspektionen und Behandlungen, z.B. mit diagnostischer und/oder therapeutischer Zielstellung, insbesondere, wenn die Abbildung mit einer Bildausgabeeinheit erfolgt.

Gemäß einer weiteren Ausführungsform wird ein Navigationsaufsatz vorgeschlagen, wobei das vordere Ende des Instruments eine Strahlungsquelle ionisierender Strahlung oder einen Ultraschallprüfkopf umfasst.

Insbesondere die Handhabung ionisierender Strahlungsquellen soll, beispielsweise im Interesse eines beabsichtigten therapeutischen Effektes, genau dosiert erfolgen. Das setzt eine genaue Platzierung der Strahlungsquelle voraus. Ein Vorteil dieser Ausführungsform besteht mithin in der Möglichkeit einer präzisen extrakorporalen oder interstitiellen oder interventionellen Brachytherapie.

Gemäß einer weiteren Ausführungsform wird ein Navigationsverfahren für ein manuell oder robotisch geführtes medizinisches Instrument vorgeschlagen. Das Navigationsverfahren umfasst die Schritte: - Bereitstellen eines Navigationsaufsatzes gemäß zumindest einer der vorstehend beschriebenen Ausführungsformen; - starres Verbinden des Navigationsaufsatzes mit dem medizinischen Instrument; - Ermitteln von zumindest drei Abstandswerten zwischen dem Navigationsaufsatz und einem Körper, zu dem das medizinische Instrument in eine vorgegebene Position gebracht werden soll; - Ermitteln aus den zumindest drei Abstandswerten von Positionsdaten, die sich auf den Körper und/oder einen seiner Bestandteile beziehen für zumindest einen Vorderabschnitt des Instruments; - Darstellen mit einer Bildausgabeeinheit des zumindest einen Vorderabschnitts in einem Abbild des Körpers und/oder eines seiner Bestandteile.

Vorteile des beschriebene Verfahrens ergeben sich aus der Möglichkeit einer bei eingeschränkter direkter visueller Kontrolle gesteigerten Präzision eines vorgenommenen Eingriffs oder einer lokal vorgenommenen Behandlung, beispielsweise einer Bestrahlung.

Gemäß einer weiteren Ausführungsform umfasst das Navigationsverfahren weiterhin das manuelle oder robotische Korrigieren einer Position des Vorderabschnitts des Instruments unter Berücksichtigung aktueller Positionsdaten (x, y, z, t), des zumindest einen Vorderabschnitts.

Mögliche Vorteile dieser Ausführungsform erschließen sich dem Fachmann unmittelbar und bestehen, beispielsweise, in der Möglichkeit einer raschen Positionskorrektur des Instruments im bzw. am Körper und der Vermeidung von unbeabsichtigten Effekten. Insbesondere kann die Berücksichtigung aktueller Positionsdaten (x, y, z) zum Zeitpunkt t das visuelle Beobachten einer bildlichen Darstellung des Instruments, beispielsweise auf einem Bildschirm oder einer Projektionsfläche, im Abbild des behandelten Körpers, oder eines Körperabschnitts umfassen.

Gemäß einer weiteren Ausführungsform umfasst das Navigationsverfahren weiterhin das Ausgeben eines Signals, sobald ein zuvor festgelegter Mindestabstand zwischen dem Vorderabschnitt des medizinischen Instruments zu einem im Abbild festlegbaren Bereich und/oder einer im Abbild festlegbaren Struktur erreicht und/oder unterschritten wird.

Vorteile dieser Ausführungsform ergeben sich aus der stetigen Kontrolle und Möglichkeit der Einhaltung eines Mindestabstandes zu von der Behandlung oder dem unmittelbaren Kontakt mit dem Instrument auszuschließenden Strukturen. Beispielsweise kann die unbeabsichtigte Schädigung oder Zerstörung von an ein Operationsgebiet anliegenden Gewebsstrukturen vermieden werden.

Die vorstehend beschriebenen Ausführungsformen können beliebig miteinander kombiniert werden. Dabei können mehrere Ausführungsformen ausgewählt und miteinander kombiniert werden. Ebenso können alle Ausführungsformen unter Weglassen einzelner oder mehrerer spezifischer Merkmale derselben miteinander kombiniert werden.

Generell wird gemäß den beschriebenen Ausführungsformen eines Navigationsaufsatzes einem Operator die ortsgenaue Applikation eines Instruments und/oder einer Sonde erleichtert und/oder eine ortsgenaue Darstellung des Instruments und/oder der Sonde, bzw. seines vorderen Abschnitts, in einer bildlichen Darstellung eines Körperabschnitts ermöglicht.

Weitere Merkmale, Aspekte und Details, die mit hier beschriebenen Ausführungsformen kombiniert werden können, werden in den abhängigen Ansprüchen, der Beschreibung und den Abbildungen offenbart.

### KURZBESCHREIBUNG DER ABBILDUNGEN

Die beiliegenden Zeichnungen veranschaulichen Ausführungsformen des vorgeschlagenen Navigationsaufsatzes und dienen zusammen mit der Beschreibung der Erläuterung der Prinzipien der Erfindung. Die Elemente der Zeichnungen sind relativ zueinander und nicht notwendigerweise maßstabsgetreu dargestellt. Gleiche Bezugszeichen bezeichnen entsprechend ähnliche Teile.

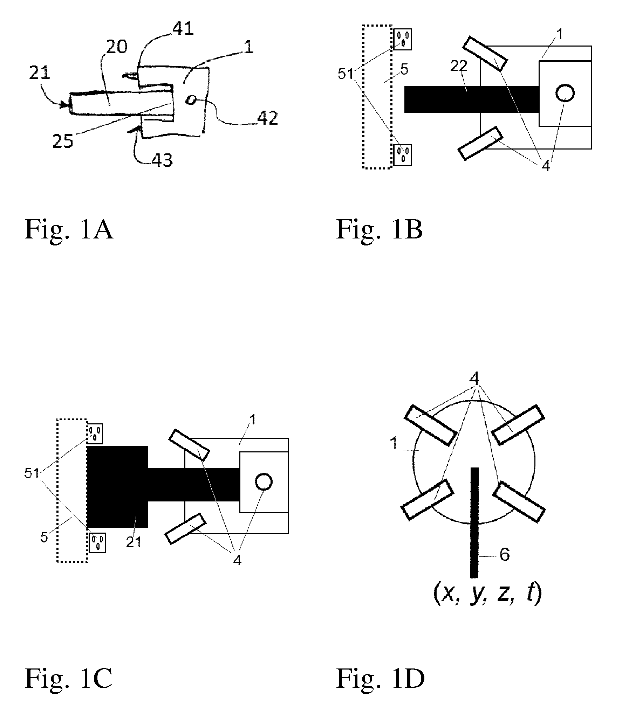

Figur 1 zeigt den Navigationsaufsatz gemäß Ausführungsbeispielen: Fig. 1A - eine skizzierte Allgemeinansicht des Navigationsaufsatzes gemäß Ausführungsbeispielen, aufgesetzt auf einem rückwärtigen Abschnitt eines medizinischen Instruments; Fig. 1B - einen Aufsatz gemäß Ausführungsbeispielen auf einem Instrument ohne direkten Körperkontakt; Fig. 1C - einen Navigationsaufsatz gemäß Ausführungsbeispielen auf einem Instrument mit Körperkontakt; 1D - eine schematische Frontansicht einer Ausführungsform gemäß Ausführungsbeispielen mit 4 Kamerasystemen.

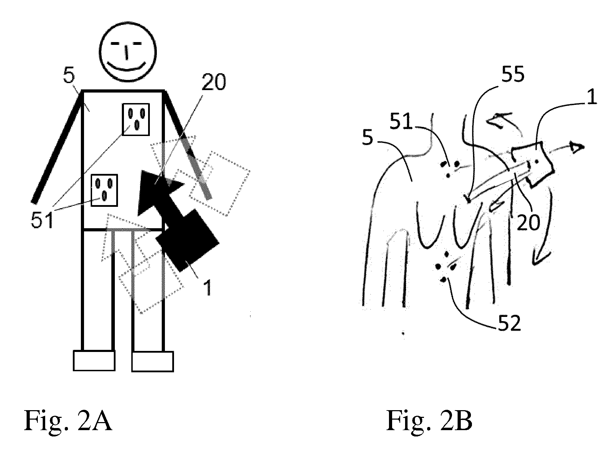

Fig. 2 veranschaulicht die Verwendung des Navigationsaufsatzes gemäß Ausführungsbeispielen gemeinsam mit einem medizinischen Instrument an einem mit Markierungen versehenen Körper.

### AUSFÜHRLICHE BESCHREIBUNG

Insbesondere ist in Fig. 1 der Navigationsaufsatz 1 mit einer das rückwärtige Ende 25 eines Instruments 20 umschließenden Ausnehmung gezeigt. Die in Fig. 1A dargestellte Ausführungsform des Navigationsaufsatzes 1 umfasst einen Navigationsaufsatz 1 mit drei Vorrichtungen zur Abstandsmessung, bzw. drei Kamerasystemen 41, 42 und 43. Das können beispielsweise Bildaufnahme-Einheiten (Digitalkamera-Bausteine) mit Autofokusfunktion sein, wobei aus zumindest drei ermittelten Werten eine aktuelle Position (x, y, z, t) des Navigationsaufsatzes 1 bezogen auf den Körper 5 bzw. bezogen zu Markierungen 51 auf der Oberfläche des Körpers 5 ermittelbar ist. Diese Möglichkeit basiert auf dem Prinzip der Triangulation. Insbesondere können unter Zugrundelegung der Linsen- bzw. Abstandsgleichung aus Bildweiten Gegenstandsweiten ermittelt werden. Erfolgt das jeweils für drei unterschiedliche Markierungspositionen 51 bzw. unterschiedliche Paarungen von Markierung und Bildaufnahmeeinheit, so ergibt sich eine eineindeutige Position des Navigationsaufsatzes 1 im Raum über dem Körper 5 (jene, rein rechnerisch mögliche, hinter bzw. im Körper liegende wird verworfen). Die Ermittlung der entsprechenden Position bzw. der entsprechenden Raumkoordinaten kann mit einem geeigneten Schaltkreis in dem Navigationsaufsatz 1 selbst erfolgen. Ebenso kann die Verarbeitung diskreter Abstandswerte auch in einer gesonderten, in den Figuren nicht gezeigten zentralen Steuer- und Verarbeitungseinheit erfolgen. Die Steuer- und Verarbeitungseinheit kann vorteilhafterweise auch die Übertragung entsprechender Signale zur Darstellung des Instruments oder seines vorderen Teils in einem bereits bestehenden Abbild des Körpers oder eines betreffenden Körperabschnitts oder eines Operations- oder Behandlungsgebietes übernehmen.

Für verschiedene Anwendungen kann eine unterschiedliche Zahl von Bildaufnahmeeinheiten 4 vorgesehen sein. So sind beispielsweise in der schematischen Skizze (Fig. 1A) drei Kamerasysteme 41, 42 und 43 jeweils so ausgerichtet, dass sie zumindest jeweils eine Markierung auf der Oberfläche des Körpers aufnehmen können. Aus den ermittelten Raumkoordinaten ergibt sich bei einem bekannten (instrumentenspezifischen) Abstand und Orientierung des Navigationsaufsatzes zu einem vorderen Ende 21 des Instruments 20 bzw. 22 eine bekannte Position des Instruments 20, 22 bezogen auf den Körper 5, bzw. auf die Körperoberfläche 5.

Fig. 1B zeigt die Anwendung des Navigationsaufsatzes 1 mit einem Instrument 22, das nicht im direkten physischen Kontakt mit dem Körper eines Patienten 5 bzw. der Körperoberfläche 5 eingesetzt wird. Ein derartiges Instrument 22 kann beispielsweise eine Gammakamera oder eine Gammasonde sein.

Fig. 1C zeigt die Anwendung des Navigationsaufsatzes 1 mit einem Instrument 21, das im direkten physischen Kontakt mit dem Körper eines Patienten 5 bzw. der Körperoberfläche 5 eingesetzt wird. Ein solches Instrument kann beispielsweise ein diagnostisches System, wie z.B. ein Ultraschallprüfkopf sein, der - beispielsweise über ein Koppelmedium vermittelt Ultraschallsignale in den Körper sendet und rücklaufende Signale auffängt und wieder in elektrische Signale wandelt (sogen. Ultraschallwandler).

Ermittelte Abstandswerte oder entsprechende Rohdaten können über ein Kabel 6 übertragen werden. Jedoch kann die Informationsübertragung (z.B. Geometrie-, Navigations-, und/oder Zeitdaten) vom Navigationsaufsatz zu einer Steuer- und Verarbeitungseinheit auch drahtlos per Funk, beispielsweise mittels "bluetooth" erfolgen. Eine Frontalansicht eines entsprechenden Navigationsaufsatzes ist in Fig. 1D schematisch gezeigt.

Die Fign. 2A und 2B zeigen unterschiedliche Zwischenpositionen (durch punktierte Linien bzw. Pfeile angedeutet) eines mit einem Navigationsaufsatz 1 versehenen Instruments 20 relativ zu fest am Körper 5 angebrachten Markierungen 51, 52. Wie erkennbar, können die Markierungen gleichartig sein 51 (vgl. Fig. 2A) oder unterschiedlich an unterschiedlichen Positionen des Körpers 51, 52 sein (vgl. Fig. 2B). Geeignete Markierungen umfassen Infrarot-Markierungen, beispielsweise IR-Sender oder -Reflektoren; einen Barcode; einen QR-Code; oder einen DataMatrix Code als Referenzbasis für die Positionsinformation, z.B. als Pflaster. Ebenso kann die Position einer Öffnung 55 in der Körperoberfläche in welche das Instrument 20 eingeführt wurde bekannt sein. Die feststehenden Markierungen 51, 52 gestatten eine Bestimmung der Position des Navigationsaufsatzes 1 über dem Körper 5. Ebenso können "Bodymarker" (wie z.B. Nase, Ohr, Augenbraue, ...) als zusätzliche Referenzmarker genutzt werden. Vorteile einer genauen Positionierung betreffen beispielsweise die exakte Positionierung einer Strahlungsquelle am genau bekannten - und ggf. synchron mit einem anderen bildgebenden Verfahren dargestellten Ort einer malignen Gewebsveränderung. Die üblicherweise genutzten elektromagnetischen Trackingverfahren decken nur einen geringen Teil des Körpers ab. So beträgt eine maximal zulässige Entfernung bei einem kommerziellen Trackingsystem (NDI) beispielsweise ca. 40 cm Abstand vom (datenverarbeitenden) Würfel. Die Dimensionen des Patientenkörpers liegen mitunter außerhalb des vom Trackingsystem charakterisierten Messvolumens. Ebenso können metallische Komponenten zu einer Änderung der Messgenauigkeit führen. Die Kameras bekannter optischer Trackingverfahren sind typischerweise sehr weit vom Patienten entfernt und lassen deswegen sehr oft den direkten Blick zum "zu trackenden" System gar nicht mehr zu. Mit einem Kameraaufsatz wie dem hier beschriebenen Navigationsaufsatz 1 können die beispielsweise beschriebenen Nachteile vermieden und zusätzliche Marker, wie die Bodymarker Nase, Ohr, Augenbraue u.a. als zusätzliche Referenzmarker genutzt werden.

Mit Hilfe der beschriebenen Ausführungsform und der angeführten Beispiele werden eingangs beschriebene Nachteile bekannter Vorrichtungen und Verfahren überwunden. Die mit etablierten Technologien erreichbare komplexe Integration elektronischer Schaltkreise und Baugruppen ermöglicht eine kompakte Bauweise des beschriebenen Navigationsaufsatzes. Beispielsweise können miniaturisierte Kamera-Module, die aus dem Bereich der Telekommunikation bekannt sind (Endgeräte) für den beschriebenen Navigationsaufsatz verwendet werden. Ebenso können angepasste Schaltkreise (z.B.: ASICs) und Komponenten mikroelektromechanischer Systeme (MEMS) wie Lagesensoren Verwendung finden. So können gemäß einer weiteren Ausführungsform Ultraschall-Abstandssensoren an Stelle der Bildaufnahmeeinheiten oder in Kombination mit solchen am Basisteil angeordnet sein. Ebenso können IR-Sensoren, elektronische (z.B. kapazitive, induktive u.a.) Abstandsmesser, Funkmodule oder geeignete optoelektronische Baugruppen zur Abstandsmessung untereinander und/oder miteinander kombiniert werden, um eine vom jeweiligen primären Darstellungssystem unabhängige oder jenes nicht störende Verfahren zur Bilderzeugung zu ermöglichen.

Während das Vorangehende auf Ausführungsformen der Erfindung gerichtet ist, können andere und weitere Ausführungsformen der Erfindung durch Kombinationen der beschriebenen aufgestellt werden, ohne vom Schutzbereich der Erfindung abzuweichen, der durch die nachfolgenden Ansprüche bestimmt wird.

## Patentansprüche

1. Navigationsaufsatz (1) für ein manuell oder robotisch geführtes Instrument (20, 22), das zur chirurgischen und/oder therapeutischen Behandlung eines Körpers (5) und/oder zur Durchführung eines Diagnostizierverfahrens am Körper (5) bestimmt ist, umfassend:
- ein Befestigungsmittel, das angepasst ist, mit dem Instrument (20, 22) starr verbindbar zu sein,
- zumindest eine Bildaufnahmeeinheit (4, 41, 42, 43), umfassend eine Vorrichtung zur Ermittlung eines Abstandswertes zwischen dem Navigationsaufsatz (1) und einer Oberfläche des Körpers (5),
wobei zumindest drei Abstandswerte zur Darstellung einer Position (x, y, z, t) des Instruments (20, 22) und/oder eines Vorderabschnitts (21) des Instruments (20, 22) bezogen auf den Körper (5) oder einen Bestandteil desselben verwendbar sind.

2. Navigationsaufsatz (1) nach Anspruch 1, wobei der Navigationsaufsatz (1) eine Ausnehmung umfasst, die so angepasst ist, dass sie reversibel und zumindest abschnittsweise formschlüssig mit einem rückwärtigen Abschnitt (25) des Instruments (20, 22) verbindbar ist, wobei die zumindest eine Bildaufnahmeeinheit (4, 41, 42, 43) benachbart zur Ausnehmung angeordnet ist.

3. Navigationsaufsatz (1) nach Anspruch 1 oder 2, umfassend eine bis sechs Bildaufnahmeeinheiten (4, 41, 42, 43), die angepasst sind, einen Abstandswert zu mindestens einem Markierungselement (51, 52) auf der Oberfläche des Körpers (5) und optional einen Wert einer axialen Rotation relativ zum Markierungselement (51, 52) zu erfassen, wenn sich das Markierungselement (51, 52) im Erfassungsbereich der Bildaufnahmeeinheit (4, 41, 42, 43) befindet.

4. Navigationsaufsatz (1) nach einem der vorstehenden Ansprüche, wobei der Navigationsaufsatz (1) angepasst ist, ermittelte Abstandswerte an eine Auswerteeinheit zu übermitteln, die in Verbindung mit einer Bildausgabeeinheit steht, wobei die Auswerteeinheit angepasst ist, sodass eine Darstellung des Instruments (20) und/oder seines Vorderabschnitts (21) mit der Bildausgabeeinheit und/oder eine Einbettung dieser Darstellung in ein bestehendes Abbild des Körpers (5) oder eines Bestandteils desselben ermöglicht wird.

5. Navigationsaufsatz (1) nach einem der vorstehenden Ansprüche, wobei der Vorderabschnitt (21) eine Strahlungsquelle ionisierender Strahlung oder einen Ultraschallprüfkopf umfasst.

6. Navigationsverfahren für ein manuell oder robotisch geführtes medizinisches Instrument (20, 21, 22) umfassend:
- Bereitstellen eines Navigationsaufsatzes (1) mit den in zumindest einem der Ansprüche 1 bis 5 beschriebenen Merkmalen;
- starres Verbinden des Navigationsaufsatzes (1) mit dem medizinischen Instrument (20, 22);
- Ermitteln von zumindest drei Abstandswerten zwischen dem Navigationsaufsatz (1) und einem Körper (5), zu dem das medizinische Instrument (20, 22) in eine vorgegebene Position gebracht werden soll;
- Ermitteln aus den zumindest drei Abstandswerten von Positionsdaten (x, y, z, t), die sich auf den Körper (5) und/oder einen seiner Bestandteile beziehen für zumindest einen Vorderabschnitt (21) des Instruments (20, 22);
- Darstellen des zumindest einen Vorderabschnitts (21) in einem Abbild des Körpers (5) und/oder eines seiner Bestandteile mit einer Bildausgabeeinheit.

7. Navigationsverfahren nach Anspruch 6, weiterhin umfassend:
- manuelles oder robotisches Korrigieren einer Position des Vorderabschnitts (21) unter Berücksichtigung aktueller Positionsdaten (x, y, z, t) des zumindest einen Vorderabschnitts (21).

8. Navigationsverfahren nach Anspruch 6 oder 7 weiter umfassend:
- Ausgeben eines Signals wenn der Vorderabschnitt 21 einen vorgebbaren Mindestabstand zu einem im Abbild festlegbaren Bereich und/oder einer im Abbild festlegbaren Struktur erreicht und/oder unterschreitet.
